# EUROPEAN PATENT SPECIFICATION

(11) **EP 0 367 354 B1**
(45) Date of publication and mention of the grant of the patent: **02.06.1993**
(21) Application number: 89202763.2
(22) Date of filing: 01.11.1989
(51) Int. Cl.: A61M 25/02

(54) **A percutaneous implant**
Perkutanes Implantat
Implant percutané

(30) Priority: 02.11.1988 NL 8802685
(43) Date of publication of application: 09.05.1990
(73) Proprietor: Stichting voor Materiaalkunde Vrije Universiteit Amsterdam "MAVU", 2333 AA Leiden (NL)
(72) Inventor: de Groot, Klaas, NL-2101 EL Heemstede (NL); Jansen, Johannes Arnoldus, NL-3144 GG Maassluis (NL)
(74) Representative: Smulders, Theodorus A.H.J., Ir.

(56) References cited:
- EP-A- 0 178 650
- EP-A- 0 194 980
- GB-A- 2 183 256

## Description

This invention relates to a percutaneous implant, comprising a subcutaneous part and a percutaneous part. Such a percutaneous implant is described in European Patent Application 0 194 980.

A percutaneous implant may be defined as an article which permanently extends through the skin via a surgically created opening. By means of the penetration through the skin a connection is established between the interior and the exterior of the body.

Percutaneous implants are often used, inter alia for transmission of energy (e.g. for an artificial heart) or information; connection of a hemodialyzer to the vascular system (kidney and peritoneal dialysis); for artificial feeding outside the esophagus; intrahepatic chemotherapy; the stimulation of muscles or parts of central nervous system; attachment of hearing aids; attachment of limbs.

Up to now the results of clinical experiments with percutaneous implants that must be able to function for a longer period of time have not been very promising. The experiments mostly failed after a period of approximately three months, inter alia owing to infection or marsupialization of the implants. Infection may be caused by influx of bacteria, debris, etc., along the implant into the tissues. Marsupialization may be caused by migration of the epidermis along the implant, resulting in a total epidermal encapsulation of the implant.

This invention is based on the insight that the clinical success of percutaneous implants depends on the formation of a stable attachment of the skin to the implant surface and that reduction of the relative movement between implant and skin increases the chance of a successful percutaneous implant.

It is known that such a reduction of the movability of the skin can be obtained by:
1) stably anchoring percutaneous implants in bone, or
2) implanting percutaneous implants in areas in which the skin is rather close to the bone surface.

However, not all of the percutaneous implants can be stabilized by means of boney tissue.

It is therefore an object of the present invention to provide a percutaneous implant capable of functioning in soft tissues for a longer period of time, also if no boney tissue is present to stabilize the percutaneous implant.

It is a particular object of the invention to provide a percutaneous implant which provides a firm base in the soft tissue that stabilizes the interface between the tissue and the implant, while at the same time is elastic so as to permit a movement of the soft tissue. Thereto, in a percutaneous implant according to the invention, the subcutaneous part comprises a part consisting of an elastic fibre mesh sheet. It is an important advantage of the percutaneous implant according to this invention that connective tissue ingrowth and vessel ingrowth may take place in the pores of the mesh sheet. This combination of connective tissue and fibre mesh sheet is so stable that the relative movement between implant and skin is reduced so that ingrowth of epithelium along the percutaneous part of the implant is avoided.On the other hand, the fibre mesh sheet is elastic so as to permit a movement of the skin. Preferably, the fibre mesh sheet is made of sintered metallic fibre. The unique property of the metallic fibre mesh sheet is the combination of the properties of metal with the properties of fibre products, resulting in that a mesh sheet made of metallic fibre is firm and porous at the same time, while permitting bending of the sheet to allow for movement of the skin. Preferably, the porosity of the fibre structure is chosen between 70 and 90 %.

Materials suitable for use as a metallic fibre in a mesh sheet according to the invention are:
- 316 stainless steel
- Inconel 601 (trademark of Inco)
- Hastalloy X (trademark of Cabot USA)
- Carpenter 20 CB 3 (trademark of Carpenters Technologies USA)
- Nichrome 80-20
- Fecralloy (trademark of UKAEA)
- Titanium
preference being given to titanium because of the excellent properties of this metal (see D.F. Williams, Biocompatibility of Clinical Implant Materials, Vol. 1, CRC Press Inc., Boca Raton, 9-44, 1981). The fact is that titanium is a very light metal having a melting point of approximately 1665 °C. The use of titanium as an implant material is known per se, inter alia for maxillofacial, oral, cardiovascular, orthopedic, and neural surgery. The use of titanium as an implant material is based on its high corrosion resistance. This corrosion resistance results from the presence of a very inert and strongly passivating layer on the metal surface. The mechanical properties of titanium may be improved by alloying, if desired; preferably, the metal employed is an alloy of titanium with 6% aluminium and 4% vanadium (Ti6Al4V).

According to the invention it is possible to allow tissue ingrowth in a fibre mesh sheet outside the patient's body. The tissue to grow in is preferably boney tissue of the patient in question. When such a fibre mesh sheet provided with ingrown tissue is used in the subcutaneous part of a percutaneous implant, its firmness is enhanced and the time required for a stable connection between the soft tissue and the implant is shortened.

It is noted that European Patent Application 0 178 650 describes a porous flexible metal fibre material for implantation to bone. The material allows bone ingrowth for stabilization of repaired bone or reconstruction of defective bone.

The invention will hereinbelow be explained with reference to the drawings, in which
Fig. 1 is a diagrammatic top view of a subcutaneous part of a percutaneous implant according to the invention;
Fig. 2 is a diagrammatic side view of a preferred embodiment of a percutaneous implant according to the invention;
Fig. 3A-D is a diagrammatic side view of a percutaneous implant according to the invention, in successive stages of the implantation;
Fig. 4 is a diagrammatic side view of a percutaneous implant according to the invention in a possible application.

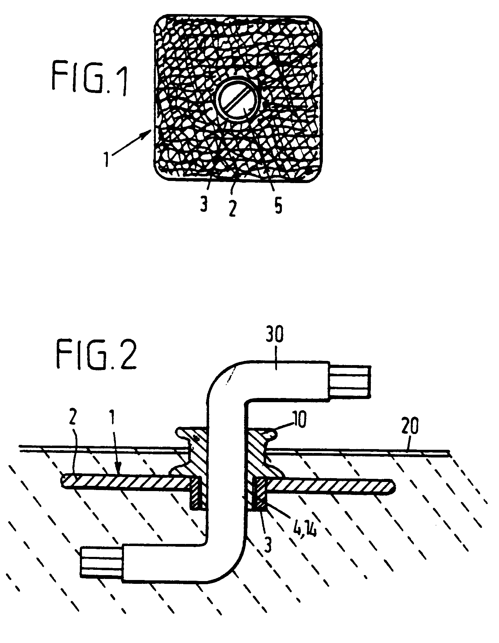

In Fig. 1 a subcutaneous part of a percutaneous implant according to the invention is generally indicated by reference numeral 1. The subcutaneous part 1 comprises a mesh sheet 2 of sintered metallic fibre.

The fibre mesh sheet successfully used was of a standard type developed for, among other things, the purposes of heat insulation,sound absorption, bone implantation, and for gas burners. The fibre diameter is preferably chosen between 2 and 25 µm, while the dimensions of the mesh sheet are preferably chosen between 12 and 15 cm.

Centrally disposed in the subcutaneous part 1 is a holding member 3 comprising a round hole provided with a screw thread 4. By means of this screw thread 4 a percutaneous part can be firmly and stably anchored in the subcutaneous part 1. When a subcutaneous part 1 is implanted, the hole provided with the screw thread 4 in the subcutaneous part 1 is closed by means of an auxiliary screw 5 so as to avoid ingrowth of tissue in this hole.

Fig. 2 is a diagrammatic representation of a percutaneous implant according to the invention, with a percutaneous part 10 anchored to a subcutaneous part 1 extending through the skin 20. The percutaneous part 10 comprises a massive or tubular structure, preferably also manufactured from the metal titanium or the alloy Ti6Al4V, and is provided with a screw thread 14 corresponding to the screw thread 4 in the subcutaneous part 2. If required, the percutaneous part 10 is provided with a hydroxylapatite layer, which is applied to the metal surface, e.g., by means of a plasma spray process.

For the percutaneous part 10 the above metals are preferred in view of their good biological and physical properties. The materials are regarded as biocompatible (see Biocompatibility of Clinical Implant Materials, Vol. 1 and 2, CRC Press Inc., Boca Raton, 1981). For these materials it is also described that hemidesmosomes may be formed in the interface between implant and skin (see J.A. Jansen, dissertation, KUN (University of Nimegen), 1984). Hemidesmosomes are regarded as specific cell structures which play a part in the adhesion of epithelium cells to a surface or to the subjacent connective tissue. The first requisite for a material suitable for a percutaneous implant is that the material can cause epithelium cells to form hemidesmosomes in the place where the skin contacts the implant surface; the above materials satisfy this requirement.

A further important advantage of titanium and Ti6Al4V over numerous other implant materials is that the former materials render it possible to manufacture the percutaneous part 10 in various forms: the form of the percutaneous part 10 in fact depends on the use of the implant. For connecting it to, e.g., a dialyzer a hollow structure will be necessary, while a substantially massive structure will suffice for the transmission of energy or information. The good physical properties of the above materials guarantee sufficient firmness of the percutaneous implant, irrespective of the form in which it is used. The hydroxylapatite coating to be applied, if desired, may ensure that the biological properties of the implant are further improved, while the physical properties are not affected.

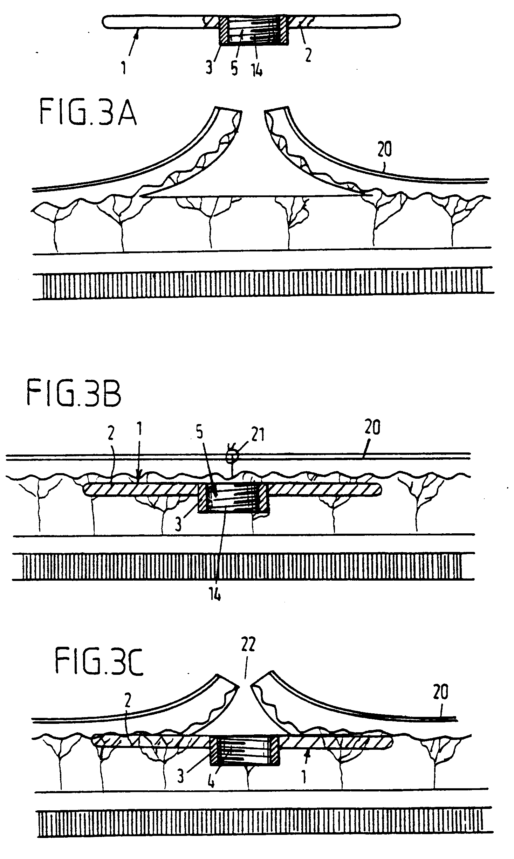

Fig. 3A shows that a first stage of the implantation procedure comprises applying the subcutaneous part 1 of the implant. The central hole in the holding member 3 of the subcutaneous part 1 of the implant is closed in this phase by means of the auxiliary screw 5 so as to avoid ingrowth of tissue in this hole.

Subsequently, the wound is closed by means of suture 21 (Fig. 3B), after which tissue ingrowth in the mesh sheet 2 of the subcutaneous part 1 may take place. After 2 to 4 months the tissue ingrowth has advanced to the extent that sufficient stability of the subcutaneous part 1 in relation to the skin 20 is obtained, while the skin remains "soft" and movable because of the elastic properties of the fibre sheet mesh.

In a second stage of the implantation procedure according to the invention (Fig. 3C) a percutaneous passage 22 is created in the skin above the central hole in the holding member 3 of the subcutaneous part 1. The auxiliary screw 5 disposed in the subcutaneous part 1 is removed and the percutaneous part 10 of the implant is fixed in the central hole in the holding member 3 of the subcutaneous part 1 by means of the screw thread 4. Subsequently, the wound is closed by applying sutures on both sides of the percutaneous part 10 (not shown), which percutaneous part 10 extends through the skin 20. During the healing process the skin 20 will adhere to the percutaneous part 10 in a manner comparable to the manner in which gums adhere to a tooth.

Percutaneous implants according to the invention are thus implanted successfully, as will be illustrated by a few examples.

### Example 1

Percutaneous implants are placed in the back of experimental animals (rabbits). After sedation of the rabbits the back is, on both sides of the spinal column, shorn, depilated, washed and disinfected with iodine. Subsequently, a longitudinal incision is made through the skin, parallel to the spinal column. The incision is about 2-3 cm in length. Then a subcutaneous pocket is created by undermining the skin with a pair of scissors. The subcutaneous part of the implant, a titanium fibre mesh sheet (in the order of 3-4 cm), is placed in this subcutaneous pocket and the wound is closed with non-resorbable sutures. After three months the skin is incised again above the subcutaneous component (incision about 1 cm in length). A percutaneous part made of hydroxylapatite is placed on the subcutaneous titanium fibre mesh sheet. Finally, the wound is closed by means of sutures.

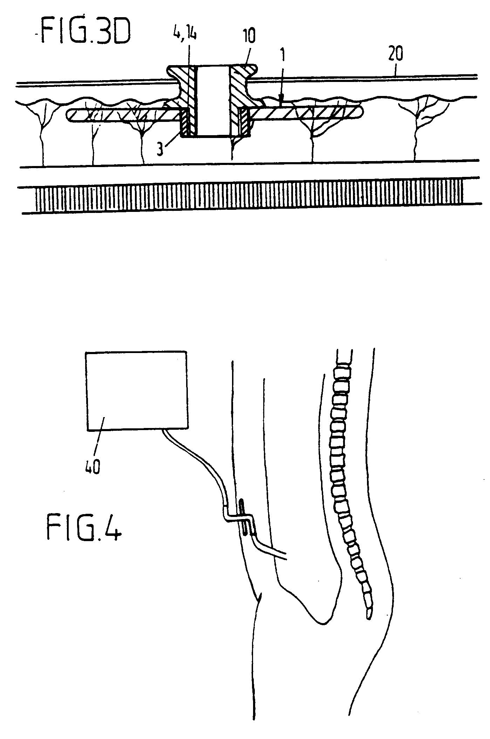

The operation procedure is diagrammatically represented by Fig. 3. The percutaneous implant remains in situ for 3-6 months. During this time the implants are clinically inspected and cleaned every week. At the end of this period the experimental animals are sacrificed and the implants with the surrounding tissues are used for histological examination (light microscopy and transmission electron microscopy). During the implantation period undisturbed wound healing was clinically observed with no sign of inflammation or migration of the skin. It was histologically established that the subcutaneous part of the implant was surrounded by a fibrous capsule. Ingrowth of connective tissue and blood vessels had taken place in the pores of the titanium mesh sheet. No inflammatory cell reactions were observed (such as plasma cells, macrophages, leukocytes). It turned out that no epidermal ingrowth had taken place along the percutaneous parts of the implant. Nor were there any signs of an inflammation reaction around the percutaneous passageway. Electron microscopic investigation shows that the adhesion of the epithelium to the implant surface is very similar to the adhesion of gingiva epithelium to tooth surface. Hemidesmosomes were observed in the interface between epithelium and implant surface.

### Example 2

One application of the invention is hemodialysis. For the purpose of this application the patient is to be provided with a device which enables the dialyzer to be connected to the vascular system of the patient. Such a device (which is diagrammatically represented by Figs. 2 and 4 ) may consist of a percutaneous implant according to the invention disposed in the patient's belly. For this application the subcutaneous part 1 of the implant measures 4x4 cm. Centrally disposed in the subcutaneous part 1 is the fastening member 3 with a threaded round hole having a diameter of 0.5-1 cm. The percutaneous part 10 of the implant is made of titanium and comprises an S-shaped hollow tube 30 which is preferably also made of titanium. The connection of the dialyzer 40 takes place by means of this hollow tube.

It will be clear to a skilled worker that it is possible to introduce amendments or adaptations in the embodiments described, without departing from the inventive concept or the scope of protection. Thus, for instance, it is possible within the scope of the invention to apply a bayonet fastening instead of the screw fastening 4, or a combination of both.

## Claims

1. A percutaneous implant, comprising a subcutaneous part (1) and a percutaneous part (10), characterized in that the subcutaneous part (1) comprises a part consisting of an elastic fibre mesh sheet (2).

2. A percutaneous implant according to claim 1, characterized in that the fibre mesh sheet (2) is made of sintered metallic fibre.

3. A percutaneous implant according to claim 1 or 2, characterized in that the porosity of the fibre mesh sheet (2) is 70-90%.

4. A percutaneous implant according to claim 2 or 3, characterized in that the metal comprises titanium.

5. A percutaneous implant according to any of claims 2 through 4, characterized in that the metal comprises the titanium alloy Ti6Al4V.

6. A percutaneous implant according to any of claims 1 through 5, characterized in that the percutaneous implant is provided with a passage (30).

7. A percutaneous implant according to any of claims 1 through 6, characterized by a holding member (3) for fixing the percutaneous part (10) relatively to the subcutaneous part (1).

8. A percutaneous implant according to claim 7, characterized in that the holding member (3) comprises a screw thread (4).

9. A device, apparently designed to function as a subcutaneous part (1) in a percutaneous implant according to any of claims 1 through 8.

10. A device according to claim 9, characterized in that the fibre mesh sheet (2) is provided with ingrown tissue.

## Patentansprüche

1. Ein perkutanes Implantat, das aus einem subkutanen Teil (1) und einem perkutanen Teil (10) besteht, dadurch gekennzeichnet, daß der subkutane Teil(1) aus einer elastischen Netzfaserfolie (2) besteht.

2. Ein perkutanes Implantat gemäß Anspruch 1, dadurch gekennzeichnet, daß die Netzfaserfolie aus einer Sintermetallfaser besteht.

3. Ein perkutanes Implantat gemäß den Ansprüchen 1 oder 2, dadurch gekennzeichnet, daß die Porösität der Netzfaserfolie (2) zwischen 70 und 90 % liegt.

4. Ein perkutanes Implantat gemäß den Ansprüchen 2 oder 3, dadurch gekennzeichnet, daß das Metall aus Titan besteht.

5. Ein perkutanes Implantat entsprechend beliebig den Ansprüchen 2 bis 4, dadurch gekennzeichnet, daß das Metall aus der Titanlegierung Ti6Al4V besteht.

6. Ein perkutanes Implantat entsprechend beliebig den Ansprüchen 1 bis 5, dadurch gekennzeichnet, daß das perkutane Implantat mit einem Durchgang (30) versehen ist.

7. Ein perkutanes Implantat entsprechend beliebig den Ansprüchen 1 bis 6, dadurch gekennzeichnet, daß eine Haltevorrichtung (3) bereitgestellt ist, die den Perkutanen Teil (10) relativ zum subkutanen Teil (1) fixiert.

8. Ein perkutanes Implantat entsprechend Anspruch 7, dadurch gekennzeichnet, daß die Haltevorrichtung ein Schraubgewinde enthält.

9. Ein Gerät, das anscheinend entworfen wurde, um die gleiche Funktion zu übernehmen, wie ein subkutaner Teil (1) in einem perkutanen Implantat, entsprechend einem der Ansprüche 1 bis 8.

10. Ein Gerät entsprechend dem Anspruch 9, dadurch gekennzeichnet, daß die Netzfaserfolie von Gewebe durchwachsen wird.

## Revendications

1. Implant percutané, comprenant une partie sous-cutanée (1) et une partie percutanée (10),
caractérisé en ce que la partie sous-cutanée (1) comprend une partie constituée d'une feuille de toile fibreuse élastique (2).

2. Implant percutané selon la revendication 1,
caractérisé en ce que la feuille de toile fibreuse (2) est réalisée en fibres métalliques agglomérées.

3. Implant percutané selon la revendication 1 ou 2,
caractérisé en ce que la porosité de la feuille de toile fibreuse (2) est de 70-90 %.

4. Implant percutané selon la revendication 2 ou 3,
caractérisé en ce que le métal comprend du titane.

5. Implant percutané selon l'une quelconque des revendications 2 à 4,
caractérisé en ce que la métal comprend l'alliage de titane Ti6Al4V.

6. Implant percutané selon l'une quelconque des revendications 1 à 5,
caractérisé en ce que l'implant percutané est muni d'un passage (30).

7. Implant percutané selon l'une quelconque des revendications 1 à 6,
caractérisé par un organe de maintien (3) pour fixer la partie percutanée (10) par rapport à la partie sous-cutanée (1).

8. Implant percutané selon la revendication 7,
caractérisé en ce que l'organe de maintien (3) comprend un filetage (4).

9. Dispositif, manifestement conçu pour fonctionner comme partie sous-cutanée (1) dans un implant percutané selon l'une quelconque des revendications 1 à 8.

10. Dispositif selon la revendication 9,
caractérisé en ce que la feuille de toile fibreuse (2) est revêtue de tissu incarné.
